# EUROPÄISCHE PATENTSCHRIFT

(11) **EP 0 426 973 B1**
(45) Veröffentlichungstag und Bekanntmachung des Hinweises auf die Patenterteilung: **10.08.1994**
(21) Anmeldenummer: 90117359.1
(22) Anmeldetag: 10.09.1990
(51) Int. Cl.: C12P 7/06, C12N 1/00

(54) **Verfahren zur Ansäuerung von Melasselösungen**
Process for the acidification of molasses solutions
Procédé pour l'acidification de solutions de mélasse

(30) Priorität: 08.11.1989 DE 3937126
(43) Veröffentlichungstag der Anmeldung: 15.05.1991
(73) Patentinhaber: DEUTSCHE HEFEWERKE GMBH, 45743 Marl (DE)
(72) Erfinder: Chiu, Chung-Wai, Dr., D-2000 Hamburg 70 (DE); Kirk, Hans-Georg, Dr., D-2000 Hamburg 70 (DE)

(56) Entgegenhaltungen:
- FR-A- 2 554 803

## Beschreibung

Die Erfindung betrifft ein neues Verfahren zum Ansäuern von verdünnten Melasselösungen auf einen pH-Wert im Bereich von 5,0 bis 5,5.

Die bei der Zuckerherstellung als Mutterlauge anfallende Melasse enthält zu etwa 50 % Zucker, 20 % Wasser und außerdem Stickstoff und Phosphor enthaltende organische und anorganische Verbindungen. Sie ist deshalb als Nährstoff für Mikroorganismen gut geeignet. Der pH-Wert der Melasse ist jedoch mit meist etwa 7 bis 9 für viele Anwendungen zu hoch.

Nach FR 2 554 803 können Zucker enthaltende Lösungen, beispielsweise Melasselösungen, mit Hilfe von Kationenaustauschern demineralisiert und dann zur fermentativen Herstellung von Alkohol oder Biogas verwendet werden.

Der anaerobe oder aerobe Stoffwechsel von Hefe ist bei einem pH-Wert zwischen 2 und 7 möglich. Üblicherweise wird für die Hefegärung jedoch ein pH-Wert von 4 bis 5,5 bevorzugt, da dieser Bereich des pH-Wertes einen Selektionsvorteil für Hefen darstellt und außerdem einen guten Infektionsschutz gegen Kontaminanten gewährleistet. Auch für die Kultivierung von Hefe für Backzwecke wird ein bestimmter saurer pH-Wert eingestellt.

Die Melasse wird deshalb nach Verdünnung mit Wasser durch Schwefelsäure auf den gewünschten pH-Wert angesäuert, bevor man sie zur Bildung von Alkohol oder zur Vermehrung von Hefe einsetzt (vgl. DRP 641 742 und L. Macher, Die Hefe, Band II (1962), 420 - 23). Man verwendet Schwefelsäure, weil sie außerordentlich preiswert ist und im Gegensatz zu anderen Säuren keinen Einfluß auf die Physiologie der Hefezellen hat. Die zugesetzte Schwefelsäure verbleibt in Form von Sulfaten am Ende der Gärung oder Hefekultivierung in der verbrauchten Nährlösung.

In verstärktem Maße werden nun Hefeabwässer anaerob gereinigt oder zur Gewinnung von Methan (Biogas) eingesetzt. Bei der Methanisierung des im Abwasser enthaltenen organischen Materials stören die Sulfate, da sie zu Schwefelwasserstoff reduziert werden und die Qualität des Biogases nachteilig beeinflussen (vgl. D. B. Archer, Enzyme Microb. Technol. 1983, 162 - 70, und G. K. Anderson et al., Process Biochemistry 1982, 28 - 32). Hohe Konzentrationen an Sulfat können sogar zu einer Hemmung der Biogasbildung führen oder diese zum Erliegen bringen. Sulfat im Abwasser kann außerdem korrosiv auf Zement- oder Betonteile wirken und zur Zerstörung von Abwassereinrichtungen beitragen. Deshalb ist es wünschenswert, Abwasser mit möglichst niedrigen Sulfatkonzentrationen für die Methanisierung einzusetzen.

Es bestand daher die Aufgabe, beim Ansäuern von Melasselösungen die Schwefelsäuremenge zu vermindern.

Überraschenderweise wird die Aufgabe dadurch gelöst, daß man die verdünnten Melasselösungen mit 100 bis 700 ml eines schwach sauren Kationenaustauschers, bezogen auf 1 kg unverdünnte Melasse, behandelt. Es war nicht zu erwarten, daß man mit relativ geringen Ionenaustauschermengen ausreichende pH-Wert-Änderungen herbeiführen kann.

Die Melasse wird zunächst durch Zugabe von Wasser vorzugsweise in eine 10- bis 70%ige wäßrige Lösung übergeführt. Dabei werden 30- bis 60%ige Melasselösungen besonders bevorzugt. Bei diesen Angaben bleiben die in der Melasse von Anfang an vorhandenen Wassergehalte unberücksichtigt.

Die verdünnten Melasselösungen werden dann mit schwach sauren Kationenaustauschern behandelt. Dabei können alle handelsüblichen, schwach sauren Kationenaustauscher zum Einsatz kommen.

Die Menge der Kationenaustauscher beläuft sich vorzugsweise auf 120 bis 300 ml, bezogen auf 1 kg unverdünnte Melasse.

Typ und Menge des verwendeten Kationenaustauschers richten sich nach dem gewünschten pH-Wert. Mit schwach sauren Kationenaustauschern in den bevorzugten Mengen lassen sich pH-Werte im Bereich von 5 bis 5,5 bequem einstellen.

Die Ansäuerung kann bei 10 bis 80 °C erfolgen, wobei Temperaturen von 20 bis 30 °C bevorzugt werden. Die Zeit, in der die Behandlung mit den Kationenaustauschern erfolgt, kann 10 Minuten oder auch 2 Stunden betragen.

Die Behandlung kann in einem Rührreaktor erfolgen. Am Ende kann hier der Kationenaustauscher abfiltriert oder die Melasselösung dekantiert werden. Man kann den Kationenaustauscher auch in eine Säule geben und dann die Melasselösung durch diese Säule leiten. Dabei kann die Behandlung diskontinuierlich und auch kontinuierlich durchgeführt werden.

Die so angesäuerten Melasselösungen können sehr gut zur Herstellung von Fermentationsprodukten eingesetzt werden. Die Melasselösungen werden im besonderen zur Herstellung von Hefemasse, beispielsweise zur Herstellung von Bäckerhefe, sowie zur Herstellung organischer Substanzen mit Hilfe von Mikroorganismen, wie zum Beispiel zur Herstellung von Alkohol durch Gärung, verwendet. Die erfindungsgemäßen Melasselösungen sind im Vergleich zu herkömmlichen, sulfatierten Melasselösungen durchaus gleichwertig. Nach beendeter Fermentation können gebrauchte Fermentationslösungen, die mit einer erfindungsgemäßen Melasselösung hergestellt worden waren, jedoch mit Vorteilen anaerob zu Biogas abgebaut werden, da eine Bildung von Schwefelwasserstoff ganz unterbleibt oder erheblich reduziert ist.

Die folgenden Beispiele sollen die Erfindung erläutern.

### Beispiel 1

In einem 6-l-Reaktor werden 2 kg Melasse, bestehend aus 25 % Rohrmelasse und 75 % Rübenmelasse, mit 2 kg Wasser verdünnt. Die Melasselösung weist einen pH-Wert von 7,40 auf. Nun werden 500 ml schwach saurer Ionenaustauscher (Typ IV, Fa. Merck, D-6100 Darmstadt) zugesetzt. Anschließend wird 30 Minuten bei 25 °C gerührt. Danach liegt der pH-Wert der Melasselösung bei 5,10.

### Beispiel 2

Es wird wie in Beispiel 1 verfahren. Es wird jedoch mit 2 kg Waschwasser vom Ionenaustauscher verdünnt.

Die Melasselösung weist nach der Ionenaustauscherbehandlung einen pH-Wert von 5,20 auf.

### Beispiel 3

3 kg Melasse von Beispiel 1 werden mit Wasser zu 6 kg Melasselösung verdünnt.

500 ml Ionenaustauscher (LEWATIT^{R} CNPLF, Fa. Bayer, D-5090 Leverkusen) werden in eine Chromatographiersäule gegeben. Anschließend wird die Melasselösung mit einer Fließgeschwindigkeit von 1,70 l/h durch die Säule geleitet. Man erhält 6 kg Melasselösung mit einem pH-Wert von 5,08.

### Beispiel 4

Es wird wie in Beispiel 3 verfahren. Es werden jedoch 3,5 kg Melasse zu 7 kg Melasselösung verdünnt und mit einer Fließgeschwindigkeit von 2,8 l/h durch die Säule geleitet. Man erhält 7 kg Melasselösung mit einem pH-Wert von 5,0.

Weitere 6 kg Melasselösung werden durch die Säule geleitet. Man erhält nun eine Lösung mit einem pH-Wert von 5,35.

### Beispiel 5

1,25 kg Rübenmelasse (aus Clauen) werden mit 1,25 kg Wasser verdünnt. Die Melasselösung hat nun einen pH-Wert von 9,2. Anschließend verfährt man wie in Beispiel 3, verwendet jedoch 500 ml Ionenaustauscher von Beispiel 1. Die Fließgeschwindigkeit beträgt 1 l/h und die Temperatur 25 °C.

Man erhält 2,5 kg Melasselösung mit einem pH-Wert von 5,0.

Weitere 3,5 kg der gleichen Melasselösung werden anschließend durch die Säule geleitet. Dabei erhält man eine Lösung mit einem pH-Wert von 5,4.

### Beispiel 6

3 kg Rübenmelasse von Beispiel 5 werden mit 3 kg Wasser verdünnt.

Man stellt dann die Bedingungen von Beispiel 5 ein, wobei man jedoch den Ionenaustauscher von Beispiel 3 verwendet.

Man erhält 6 kg Melasselösung mit einem pH-Wert von 4,9.

### Beispiel 7

1,5 kg Rübenmelasse (aus Belgien) werden mit 1,5 kg Wasser verdünnt. Die so erhaltene Melasselösung hat einen pH-Wert von 8,0.

Man verfährt wie in Beispiel 5. Dabei erhält man 3 kg Lösung mit einem pH-Wert von 5,0.

Anschließend werden noch weitere 3 kg Melasselösung durch die Säule geleitet. Man erhält nun eine Lösung mit einem pH-Wert von 5,3.

### Beispiel 8

Beispiel 6 wird wiederholt mit der Änderung, daß man Rübenmelasse von Beispiel 7 einsetzt.

Man erhält 6 kg Melasselösung mit einem pH-Wert von 4,7.

### Beispiel 9

Melasse aus 25 % Rohrmelasse und 75 % Rübenmelasse wird mit der gleichen Menge Wasser verdünnt. Der pH-Wert der Melasselösung liegt bei 7,2.

Man verfährt wir in Beispiel 6. Nach einem Durchsatz von 6,9 kg liegt der pH-Wert bei 4,7. Nach einem Durchsatz von 8,8 kg wird ein pH-Wert von 5,0 gemessen.

### Beispiel 10

### Herstellung von Backhefe

5 kg Melasselösung mit einem pH-Wert von 5,0 werden gemäß Beispiel 4 werden hergestellt.

In einem 20-l-Fermenter werden 21 g NH₄Cl und 10 ml konzentrierte H₃PO₄ mit 10 l Wasser versetzt und mit 400 g Stellhefe (Stamm DHW S-3, Fa. Deutsche Hefewerke, D-4370 Marl) angestellt. Es wird bei 30 °C aerob unter Melassezulauf fermentiert. Der pH-Wert wird durch Zudosierung von Ammoniaklösung bei 5,0 konstant gehalten. Nach einer Fermentationszeit von 24 Stunden werden etwa 2 400 g Backhefe (43 bis 47 % Protein, helle Farbe) geerntet. Die so hergestellt Hefe entspricht qualitativ einer Backhefe, die mit durch Schwefelsäure angesäuerter Melasselösung fermentiert wird. (Die Hefemenge-Angaben beziehen sich auf 27 % Trockensubstanz).

### Beispiel 11

### Herstellung von Alkohol

In einem 20-l-Fermenter werden 6 kg Melasselösung von Beispiel 3 mit 4 kg Wasser verdünnt, worauf mit NH₃-Wasser auf pH 5,5 eingestellt wird. Man setzt 200 ml Nährsalzlösung, die 25 g/l KH₂PO₄ und 50 g/l MgSO₄ enthält, sowie 100 g Saccharomyces cerevisiae (27 % Trockensubstanz, Stamm DHW S-3, Fa. Deutsche Hefewerke, D-4370 Marl) zu, worauf bei 30 °C und pH 5,5 anaerob 30 Stunden fermentiert wird.

Nach dieser Zeit beträgt die Alkoholkonzentration 9,45 Vol.-%, was einer Ausbeute von 750 g Alkohol entspricht.

## Patentansprüche

1. Verfahren zum Ansäuern von verdünnten Melasselösungen auf einen pH-Wert von 5,0 bis 5,5,
dadurch gekennzeichnet,
daß man die Melasselösungen mit 100 bis 700 ml eines schwach sauren Kationenaustauschers, bezogen auf 1 kg unverdünnte Melasse, behandelt.

2. Verfahren nach Anspruch 1,
dadurch gekennzeichnet, man 10- bis 70%ige Melasselösungen einsetzt.

3. Verfahren nach Anspruch 1,
dadurch gekennzeichnet,
daß man 120 bis 300 ml Kationenaustauscher pro kg unverdünnte Melasse verwendet.

## Claims

1. A process for acidifying diluted molasses solutions to a pH of from 5.0 to 5.5, characterized in that the molasses solutions are treated with from 100 to 700 ml of a weakly acidic cation exchanger, based on 1 kg of undiluted molasses.

2. A process according to claim 1, characterized in that from 10 to 70 % strength molasses solutions are used.

3. A process according to claim 1, characterized in that from 120 to 300 ml of cation exchanger is used per kg of undiluted molasses.

## Revendications

1. Procédé pour acidifier, à une valeur de pH de 5,0 à 5,5, des solutions de mélasse diluées,
caractérisé par le fait que l'on traite les solutions de mélasse par 100 à 700 ml d'un échangeur de cations faiblement acide, relativement à 1 kg de mélasse non diluée.

2. Procédé selon la revendication 1,
caractérisé par le fait que l'on utilise des solutions de mélasse à 10-70 %.

3. Procédé selon la revendication 1,
caractérisé par le fait que l'on utilise de 120 à 300 ml d'échangeur de cations par kg de mélasse non diluée.
